# EUROPEAN PATENT APPLICATION

(11) **EP 3 628 663 A1**
(43) Date of publication of application: **01.04.2020**
(21) Application number: 18306257.9
(22) Date of filing: 26.09.2018
(51) Int. Cl.: C07D 233/60, A61K 31/4164, A61P 31/12

(54) **ANTIVIRAL COMPOUNDS**

(71) Applicant: UNIVERSITE DE BORDEAUX, 33000 Bordeaux (FR); INSTITUT NATIONAL DE LA SANTE ET DE LA RECHERCHE MEDICALE (INSERM), 75013 Paris (FR); Centre National de la Recherche Scientifique (CNRS), 75016 Paris (FR); Université de Rennes 1, 35000 Rennes (FR)
(72) Inventor: WODRICH, Harald, 33400 Talence (FR); CHEVET, Eric, 35700 Rennes (FR); VAN DE WEGHE, Pierre, 35770 Vern sur Seiche (FR); JEAN, Mickael, 22400 Lamballe (FR)
(74) Representative: Nony

(57) **Abstract**

The present invention relates to a compound of formula (I): 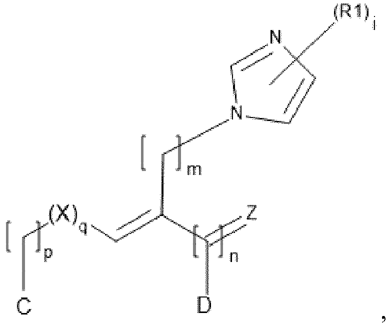 to compositions comprising it and to their use in the prevention and/or treatment of viral infections, and in particular by a non-enveloped DNA virus and/or an enveloped RNA virus.

The inventors found that the composed of formulae (I), (II) and (III), as defined herein, represent a novel class of antiviral agents with broad applicability against a wide spectrum of viruses.

SEQUENCE LISTING

<110> Universite de Bordeaux
      Institut National de la Sante et de la Recherche Medicale
      (INSERM)
      Centre National de la Recherche Scientifique (CNRS)
      Universite de Rennes 1

<120> NOVEL ANTIVIRAL COMPOUNDS

<130> PR81251

<160> 2

<170> BiSSAP 1.3.6

<210> 1
<211> 45
<212> PRT
<213> Artificial Sequence


<220>
<223> WW3_Nedd.4.1

<400> 1
Pro Ser Glu Ile Glu Gln Gly Phe Leu Pro Lys Gly Trp Glu Val Arg
1               5                   10                  15
His Ala Pro Asn Gly Arg Pro Phe Phe Ile Asp His Asn Thr Lys Thr
            20                  25                  30
Thr Thr Trp Glu Asp Pro Arg Leu Lys Ile Pro Ala His
        35                  40                  45

<210> 2
<211> 44
<212> PRT
<213> Artificial Sequence


<220>
<223> WW3_Nedd.4.2 peptide

<400> 2
Gly Ala Met Glu Gln Ser Phe Leu Pro Pro Gly Trp Glu Met Arg Ile
1               5                   10                  15
Ala Pro Asn Gly Arg Pro Phe Phe Ile Asp His Asn Thr Lys Thr Thr
            20                  25                  30
Thr Trp Glu Asp Pro Arg Leu Lys Phe Pro Val His
        35                  40

## Description

### FIELD OF THE INVENTION

The present application relates to antiviral compounds and to their use for the treatment and/or prevention of viral infections.

### BACKGROUND OF THE INVENTION

Viruses are the causative agent in a wide variety of human infectious diseases.

Efforts to treat or prevent viral infection are generally classified into two broad categories: vaccines and antiviral drugs.

Antiviral medications are advantageously able to treat individuals who are already infected as well as preventing viral diseases where vaccination methods are seen as unavailable or unlikely.

Illustratively WO 03/063869 identifies a set of compounds altering the permeability of ion channels, which are effective in controlling the replication and/or spread of viruses belonging the Picornaviridae family (non-enveloped RNA viruses).

Accordingly, there remains a constant need to widen the therapeutic arsenal to treat and/or prevent viral diseases. This is more particularly important in view of the increasing resistance of many viruses to already known antiviral drugs.

Moreover, despite noted success in the design and development of novel antiviral molecules in recent years, existing therapies are limited in terms of the number and breadth of viruses that they may be used to treat.

Indeed, one of the main problems associated with an antiviral agent having a wide spectrum of action is that it is often toxic for the individual to which these molecules are administered.

There is thus a need for novel antiviral compounds able to prevent and/or treat viral infections. Thus, there is also a need for antiviral compounds able to prevent and/or treat a broad set of viral infections, and in particular viral infections by non-enveloped DNA viruses and enveloped RNA viruses.

There is also a need for antiviral compounds able to inhibit the propagation of a virus, to inhibit the egress of a virus from the infected cells, to inhibit the replication of a virus in cells infected by said virus and/or to reduce the nuclear accumulation of viral genomes in cells infected by said virus.

There is furthermore a need for antiviral compounds capable of targeting a variety of virus types without being toxic for the individual to which these molecules are administered.

There is also a need for antiviral compounds having a better antiviral activity than antiviral treatments already available in clinics.

### SUMMARY OF THE INVENTION

The present invention aims to meet the here-above indicated needs.

According to the inventors' experimental results, the presently claimed compounds, defined as indicated here-after, are surprisingly able to prevent and/or treat viral infections. In particular, these compounds are able to act against a wide spectrum of viruses, and in particular against viruses as different as non-enveloped DNA viruses, such as adenoviruses, and enveloped RNA viruses, such as filoviruses.

Accordingly, one of the objects of the present invention relates to a compound of formula (I): 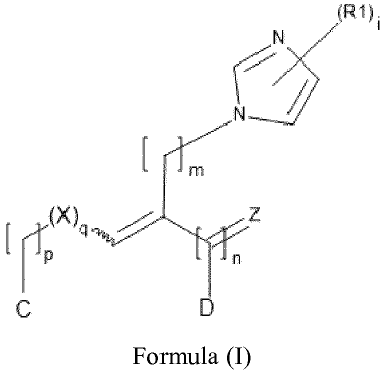 wherein:
q is 0 or 1;
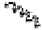 represents a single bond;
X, when q is 1, is selected from the group consisting of -S-, -O-, -C(O)- and -CH₂-;
C and D, independently, represent a non-substituted aromatic ring or an aromatic ring substituted by one or more substituents independently selected from the group consisting of a halogen atom, a C₁-C₄ alkyl group, a C₁-C₄ alkoxy group, a hydroxyl group, a -NO₂ group, a -NR₂R₃ group and a -CN group, with R₂ and R₃ being independently selected from a hydrogen atom and a C₁-C₄ alkyl group;
Z represents O or S;
m and p, independently, are 0, 1 or 2;
n is 0 or 1;
i is 0, 1, 2 or 3; and
R1 independently represents a halogen atom or a C₁-C₄ alkyl group;
or one of its pharmaceutically acceptable salts;
said compound of formula (I) being in all the possible racemic, enantiomeric and diastereoisomeric isomer forms.

In a preferred embodiment, the compound of formula (I) according to the present invention is a compound of formula (II): 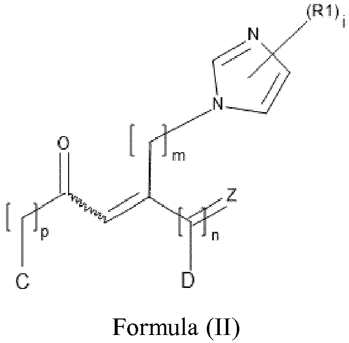
wherein C, D, Z, 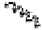 , m, n, p, i and R1 are as defined here-above;
or one of its pharmaceutically acceptable salts;
said compound of formula (II) being in all the possible racemic, enantiomeric and diastereoisomeric isomer forms.

In a particular embodiment, the compound of the invention is of formula (III): 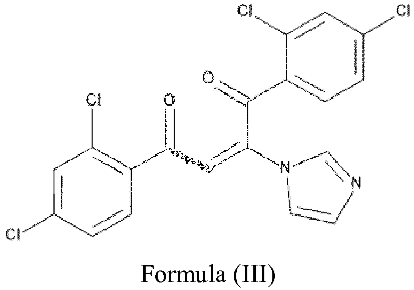
wherein 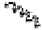 represents a single bond;
or one of its pharmaceutically acceptable salts;
said compound of formula (III) being in all the possible stereoisomeric forms. In a particular embodiment, the compound of formula (III) is of formula (IIIa):

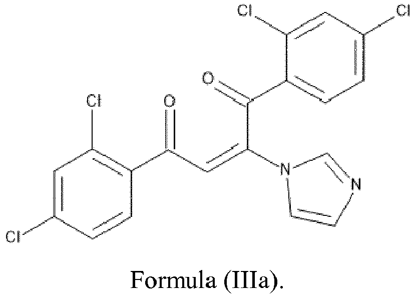

In a particular embodiment, a compound according to the invention, i.e. a compound of formula (I), (II) or (III), can be comprised in a composition comprising a pharmaceutically acceptable carrier, preferably in an intravenous composition.

Accordingly, another object of the present invention is a composition comprising, in a pharmaceutically acceptable carrier, at least one compound of formula (I), (II) or (III) of the invention.

The present application further relates to a compound according to the invention for its use in the prevention and/or treatment of viral infections by a non-enveloped DNA virus and/or an enveloped RNA virus, in particular by an adenovirus and/or a filovirus, more particularly by a non-enveloped virus, in particular by an adenovirus.

The present application also relates to a composition according to the invention for its use in the prevention and/or treatment of viral infections by a non-enveloped DNA virus and/or an enveloped RNA virus, in particular by an adenovirus and/or a filovirus, more particularly by an adenovirus.

Compounds according to the invention notably possess the following advantageous properties:
(i) they possess an antiviral activity, in particular against very different viruses such as non-enveloped DNA viruses, for example adenoviruses, and enveloped RNA viruses, for example filoviruses;
(ii) they are able to inhibit the propagation of a virus;
(iii) they are able to inhibit the replication of a virus in infected cells;
(iv) they are able to reduce the nuclear accumulation of viral genomes in cells infected by said virus; and
(v) they are not toxic.

The compounds of the present invention moreover provide the advantage of being more efficient than a standard clinical antiviral drug: Cidofovir™, as demonstrated in the following examples.

### FIGURE LEGENDS

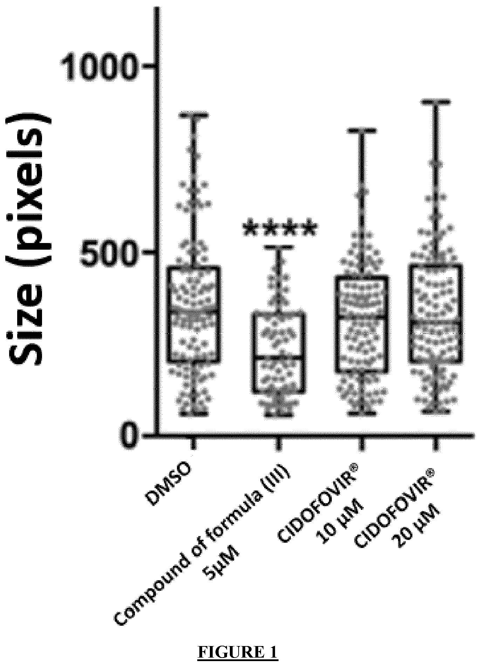

**Figure 1****:** Effect of the compound of formula (IIIa) on adenovirus spread. The figure represents the effect of DMSO or of the compound of formula (IIIa) (5µM) on adenovirus spreading in HEK293 cells infected with recombinant E1-deficient GFP expressing HAd-C5-GFP at 0.1 physical particle per cell for 24 hours followed by inoculums removal and overlay with agarose supplemented with 5µM of DMSO (negative control) or compound of formula (IIIa). Plaque sizes were measured after 6 days post infection. The mean size was determined from about 30 plaques derived from three independent experiments. Abscissa: compound administered. Ordinate: Plaque size (pixel). (****P<0.0001)
   Cidofovir™ was also tested at 10 µM or 20 µM. Plaque sizes were determined and are shown as box-plots (n>74).
   Abscissa: compound administered. Ordinate: Plaque size (pixel). (**P<0.01) (****P<0.0001)
**Figure 2****:** Toxicity measure of the compound of formula (IIIa) on U2OS cells.
Abscissa: Compound of formula (IIIa) concentration (µM). Ordinate: Normalized cell viability (%).
**Figure 3****:** represents the binding ability of the compound of formula (IIIa) to the WW3 peptide target domain WW3-NEDD4.1, compared to the one of imidazole (control).
   Abscissa: concentration titrant (nM). Ordinate: fraction bound.
**Figure 4**: represents the binding ability of the compound of formula (IIIa) to the WW3 peptide target domain WW3-NEDD4.2.
   Abscissa: concentration titrant (nM). Ordinate: fraction bound.
**Figure 5**: represents a photograph of a western blot analysis of affinity matrix (RFP-trap) isolated RFP-tagged protein VI WT (including the PPxY targeted by the NEDD4.2 WW domain which itself is targeted by the compound) or protein VI M1 (with the PPxY compound targeted by the NEDD4.2 WW domain mutated to PGAA), from cells treated with increasing amounts (0, 2, 5, 10, 25 µM) of the compound of formula (IIIa) for 2h, using anti-ubiquitin antibodies and anti-protein VI antibodies, and shows compound concentration and PPxY motif dependent ubiquitylation of protein VI.
**Figure 6****:** represents photographs of western blots analysis using anti protein VI antibodies of purified virus particles from which protein VI has been released by mild heat treatment (+48°C) followed by *in vitro* ubiquitylation reactions supplemented with recombinant NEDD4.2 and DMSO (-) or two concentrations of the compound of formula (IIIa) and carried out for 1h at 30°C or left on ice.
**Figure 7****:** represents photographs of fluorescence microscopy showing the expressing levels and positions of GFP-NEDD4.1 or GFP-NEDD4.2 generated through single copy flp-FRT integration and treated with the compound of formula (IIIa) or the control DMSO.
**Figure 8****:** represents the quantification of AdV positive for GFP-NEDD4.2 expressing cells infected in absence or presence of 10µM of the compound of formula (IIIa) with fluorescently labelled viruses and association between viruses and NEDD4.2-GFP, using fluorescence microscopy.
   Abscissa: AdV positive for NEDD4.2 (%). Ordinate: time post infection (minutes).
**Figure 9****:** represents lists of proteins with significant changes in ubiquitylation upon treatment of U2OS cells with the compound of formula (IIIa).
   The two left side columns entitled with "UP" are lists of proteins with increased ubiquitylation at the indicated concentrations and the two right side columns entitled with "DOWN" are lists of proteins with decreased ubiquitylation at he indicated concentrations.
   The diagram shows the overlap in increased and decreased ubiquitylation between the two tested concentrations.

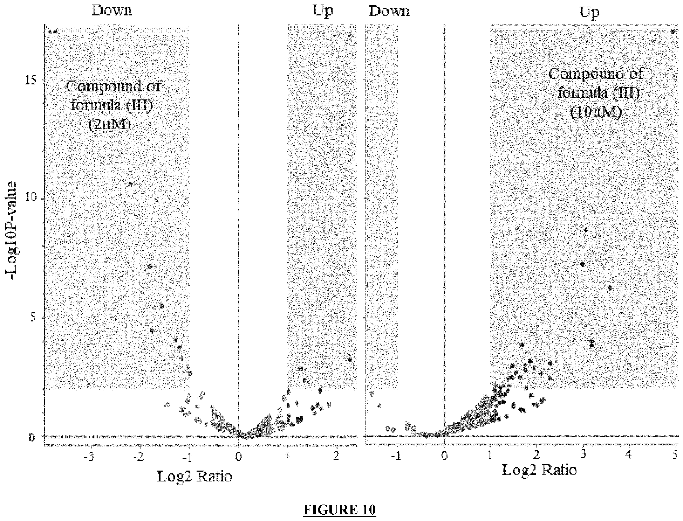

**Figure 10**: represents a volcano diagram showing the significant changes of individual ubiquitylation identified from total cellular protein extracts at two different concentrations of the compound of formula (IIIa). The down parts of the graphs depict reduced ubiquitylation and the up parts depict an increase in ubiquitylation.
   Abscissa: fold change (compared to non-treated control). Ordinate: negative log of the p-value (indicating statistical significance).

### DETAILED DESCRIPTION OF THE INVENTION

### Definitions

As used herein:
- "prevention" or "prevent" means at least partly reducing the risk of manifestation of a given phenomenon, i.e., in the present invention, a viral infection of an individual. A partial reduction implies that the risk remains, but to a lesser extent than before implementing the invention. Thus, "prevention" may also encompass the reduction of likelihood of manifestation of a given phenomenon, i.e., in the present invention, a viral infection of an individual.
- "inhibition" or "inhibit" means totally or partially reducing the manifestation of a given phenomenon, i.e., in the present invention, a viral infection, the propagation of a viral infection, the replication of a virus in infected cells and/or the nuclear accumulation of viral genomes in cells infected with a virus. Accordingly, inhibition may also refer to a reduction in viral load and/or pathogenicity.
- "pharmaceutically acceptable carrier" refers to a carrier medium which does not interfere with the effectiveness of the biological activity of the active ingredient(s) and which is not excessively toxic to the host at the concentration at which it is administered. The use of such media for pharmaceutically active substances is well known in the art (see for example "Remington's Pharmaceutical Sciences", E. W. Martin, 18th Ed., 1990, Mack Publishing Co.: Easton, Pa.).
- "individual" is intended for an animal, including human beings, affected or likely to be affected with a virus infection according to the invention. Said animal can in particular be intended for livestock, such as cattle, pigs and poultry; other non-human mammals such as pet, zoo or sports animals ; or human beings, affected or likely to be affected with a virus infection according to the invention. Said individual is preferably a human being.
- "viral infection" and "infected with a virus" as used herein mean that said human or non-human has been exposed to a pathogenic virus RNA or DNA and that said virus is attached to one or more cells of the host then penetrated (or may enter) into said one or more cells and have (or may have) detrimental effects for at least one cell of said animal or human.

In particular such viral infection is able to evolve towards clinical signs or accompanying said infection induced pathologies. Thus, a "viral infection" within the meaning of the present invention includes both the earliest phases of viral contamination, as well as the later phases and intermediate phases of viral contamination.
- "pharmaceutically acceptable salts" includes salts of compounds of the present invention derived from the combination of such compounds with non-toxic acid or base addition salts.

Acid addition salts include inorganic acids such as hydrochloric, hydrobromic, hydroiodic, sulfuric, nitric and phosphoric acid, as well as organic acids such as acetic, citric, propionic, tartaric, glutamic, salicylic, oxalic, methanesulfonic, para-toluenesulfonic, succinic, and benzoic acid, and related inorganic and organic acids.

Base addition salts include those derived from inorganic bases such as ammonium and alkali and alkaline earth metal hydroxides, carbonates, bicarbonates, and the like, as well as salts derived from basic organic amines such as aliphatic and aromatic amines, aliphatic diamines, hydroxy alkamines, and the like. Such bases useful in preparing the salts of this invention thus include ammonium hydroxide, potassium carbonate, sodium bicarbonate, calcium hydroxide, methylamine, diethylamine, ethylenediamine, cyclohexylamine, ethanolamine and the like.

The pharmaceutically acceptable salts of compounds of the present invention can also exist as various solvates, such as with water, methanol, ethanol, dimethylformamide, ethyl acetate and the like. Mixtures of such solvates can also be prepared. The source of such solvate can be from the solvent of crystallization, inherent in the solvent of preparation or crystallization, or adventitious to such solvent. Such solvates are within the scope of the present invention.

In the context of the present, invention the terms below have the following meanings:
- a halogen atom: a fluorine, a chlorine, a bromine or an iodine;
- Ct-Cz: a carbon chain that can have from t to z carbon atoms, where t and z may have the values from 1 to 7; for example, C₁-C₄ is a carbon chain that may have from 1 to 4 carbon atoms;
- C₁-C₄ alkyl as used herein respectively refers to C₁-C₄ normal, secondary or tertiary saturated hydrocarbon. Non limiting examples are methyl, ethyl, propyl, isopropyl, butyl, isobutyl or tertbutyl;
- C₁-C₄ alkoxy is intended to mean an -O-(C₁-C₄)alkyl radical where the C₁-C₄ alkyl group is as defined above. Non limiting examples are methoxy, ethoxy, propoxy, isopropoxy, butoxy, sec-butoxy or tert-butoxy.

The present inventors have performed a huge amount of work with the view of identifying novel substances endowed with antiviral properties. In particular, the inventors managed to unexpectedly identify compounds of formula (I) according to the invention having a wide antiviral scope of action and not toxic for the individual to which they are administered. Even more surprisingly, the compounds of the present invention have a better antiviral activity than a standard clinical antiviral drug: Cidofovir™.

### Compounds of the invention

The inventors have unexpectedly found that the compounds of the present invention possess an antiviral activity, and more importantly an antiviral activity against a wide spectrum of viruses, including both non-enveloped DNA viruses, such as adenoviruses, and enveloped RNA viruses, such as filoviruses.

Without wishing to be bound by the theory, the inventors are of the opinion that the compounds of the invention behave as antiviral agents by perturbing the interaction between NEDD4 ubiquitin ligases and viral [LP]PxY motifs. As illustrated in the examples, a compound according to the invention is indeed able to either increase or decrease the ubiquitylation of cellular proteins, depending of their concentration. For example, the compounds of formula (III) according to the invention favors deubiquitylation when used in a low concentration, such as 2µM, and favors cellular proteins ubiquitylation when used in higher concentrations, such as 10µM.

NEDD4 ubiquitin ligases of the HECT-E3 family are evolutionary conserved in Eukaryotes. Also, several virus families contain [LP]PxY peptide motifs embedded in their genome-encoded proteins. Viral [LP]PxY motifs belong to a group of short viral peptide motifs historically named *late domains* due to their prominent role at late stages in the lifecycle of several enveloped viruses.

Accordingly, the inventors are of the opinion that the compounds of the invention represent a novel class of antiviral agents with broad applicability.

Accordingly, the present invention relates to a compound of formula (I) as defined here-after and to for its use in the prevention and/or treatment of viral infections.

The compounds of formula (I) according to the invention are as follows: 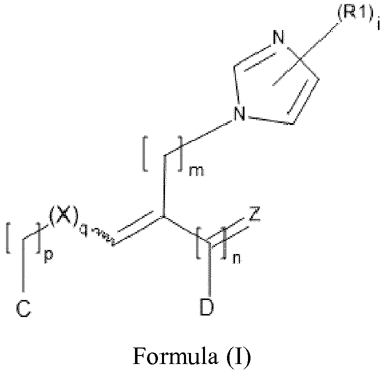 wherein:
q is 0 or 1;
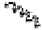 represents a single bond; X, when q is 1, is selected from the group consisting of -S-, -O-, -C(O)- and -CH₂-;
C and D, independently, represent a non-substituted aromatic ring or an aromatic ring substituted by one or more substituents independently selected from the group consisting of a halogen atom, a C₁-C₄ alkyl group, a C₁-C₄ alkoxy group, a hydroxyl group, a -NO₂ group, a -NR₂R₃ group and a -CN group, with R₂ and R₃ being independently selected from a hydrogen atom and a C₁-C₄ alkyl group;
Z represents O or S;
m and p, independently, are 0, 1 or 2;
n is 0 or 1;
i is 0, 1, 2 or 3; and
R1 independently represents a halogen atom or a C₁-C₄ alkyl group;
or one of its pharmaceutically acceptable salts.

The compounds of formula (I) according to the invention can be in all the possible racemic, enantiomeric and diastereoisomeric isomer forms.

As indicated previously, 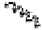 represents a single bond and materializes the possibility for the double bond of the compounds of the invention to be in the Z (cis) or E (trans) form. In a particular embodiment, a compound of formula (I) of the invention is preferably in the E form (i.e. in the trans form).

In a particular embodiment, q is 1.

In a particular embodiment, q is 1 and X represents a -C(O)- group.

In a particular embodiment, C and D, independently, represent a phenyl ring, in particular a phenyl ring substituted with one or two halogen atom(s), preferably one or two chlorine atom(s).

In a particular embodiment, C and D are different. According to this embodiment, C or D can represent a phenyl ring substituted with two chlorine atoms while the other one from C and D represents a phenyl ring substituted with one chlorine atom. According to this embodiment, D can represent a phenyl ring substituted with two chlorine atoms and C can represent a phenyl ring substituted with one chlorine atom.

In a preferred embodiment, C and D are identical. According to this embodiment, C and D both preferably represent a phenyl ring substituted with two chlorine atoms.

In a particular embodiment, m and p are independently 0 or 1.

m is preferably 0 or 1, and is in particular 0.

p is preferably 0 or 1, and is in particular 0.

In a particular embodiment, m and p are both 0.

In a particular embodiment, n is 1.

In a particular embodiment, Z represents O.

In an embodiment of the invention, n is 1 and Z represents O.

In a preferred embodiment, i is 0.

In a preferred embodiment, a compound of formula (I) according to the present invention is a compound of formula (II): 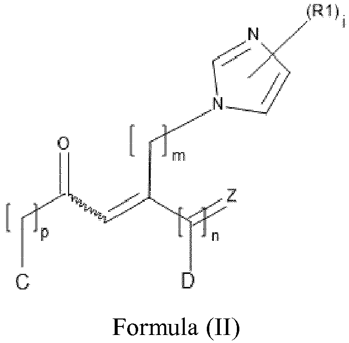
wherein C, D, Z, 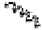 , m, n, p, i and R1 are as defined previously;
or one of its pharmaceutically acceptable salts,
said compound of formula (II) being in all the possible racemic, enantiomeric and diastereoisomeric isomer forms.

In particular, a compound of formula (II) described here-above is preferably such that C and D independently represent substituted phenyl rings.

According to a particular embodiment, C and D of a compound of formula (II), independently, represent substituted phenyl rings with at least one halogen atom, preferably 1 or 2 halogen atom(s), more preferably 1 or 2 chlorine atom(s).

In a preferred embodiment, C and D of a compound of formula (II) are identical. According to this embodiment, C and D of a compound of formula (II) can both preferably represent a phenyl ring substituted with two chlorine atoms.

m of a compound of formula (II) is preferably 0 or 1, and is in particular 0.

p of a compound of formula (II) is preferably 0 or 1, and is in particular 0.

In a particular embodiment, m and p of a compound of formula (II) are both 0. In a particular embodiment, n is 1 in a compound of formula (II).

In a particular embodiment, Z represents O in a compound of formula (II).

In an embodiment of the invention, n is 1 and Z represents O.

In a preferred embodiment, i is 0.

According to a particularly preferred embodiment, a compound of formula (I) or (II) of the invention is a compound of formula (III): 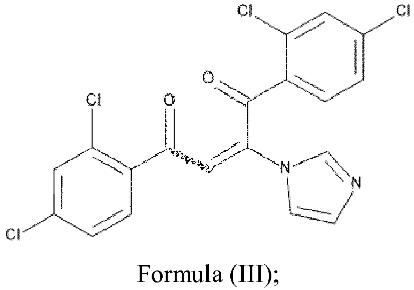
wherein 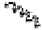 represents a single bond;
said compound being in all the possible stereoisomeric forms.

As mentioned above, a compound of formula (III) can be in the Z (cis) or E (trans) form.

In a preferred embodiment, the compound of formula (III) is preferably in the E form (i.e. in the trans form), and is thereof the compound of formula (IIIa):

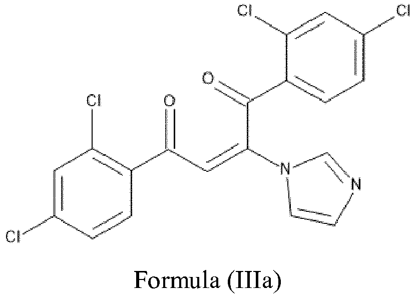

### Preparation of the compounds of formulae (I) and (II) of the invention

The compounds of formula (I) of the present invention may be prepared in a number of methods well known to those skilled in the art, including, but not limited to those described below, or through modifications of these methods by applying standard techniques known to those skilled in the art of organic synthesis.

They notably can be prepared according to the various methods disclosed in Yuki Matsuoka et al. (Tetrahedron 62(34) (2006) 8199-8206), in Ravi Shankar et al. (Tetrahedron Letters 47(18) (2006) 3077-3079), in Kashima C. et al. (Heterocycles, Vol. 15, No. 2, (1981) 961-964) and in Kashima C. et al. (J. Heterocyclic Chem., 24, 1595-6 (1987)).

The compounds of formula (II) of the present invention may be prepared on the basis of these methods as they are included in the general formula (I).

The compounds of formula (III), and in particular the compound of formula (IIIa), which are included in the general formulae (I) and (II), can be prepared on the basis of the method described here-after according the various methods disclosed in I. Yavari et al. (Tetrahedron Letters 43 (2002), 9449-9452) and in C. Kashima et al. (Synthesis (1980), 880-881), .

### Application

Compounds of formulae (I), (II) and (III) according to the invention can in particular be used in the prevention and/or treatment of viral infections.

Viruses more particularly considered in the present invention are non-enveloped DNA viruses and/or enveloped RNA viruses, and in particular are non-enveloped DNA viruses.

Non-enveloped DNA viruses can in particular be selected from the group consisting of Papillomaviridae viruses, Polyomaviridae viruses, Adenoviridae viruses, Parvoviridae viruses, Reoviridae viruses, Hepevirus, Norovirus, and Enterovirus.

Papillomaviridae viruses can in particular be selected from the group consisting of Alphapapillomavirus, Betapapillomavirus, Deltapapillomavirus, Dyodeltapapillomavirus, Dyoepsilonpapillomavirus, Dyoetapapillomavirus, Dyoiotapapillomavirus, Dyokappapapillomavirus, Dyolambdapapillomavirus, Dyomupapillomavirus, Dyonupapillomavirus, Dyoomikronpapillomavirus, Dyopipapillomavirus, Dyorhopapillomavirus, Dyosigmapapillomavirus, Dyothetapapillomavirus, Dyoxipapillomavirus, Dyozetapapillomavirus, Epsilonpapillomavirus, Etapapillomavirus, Gammapapillomavirus, Iotapapillomavirus, Kappapapillomavirus, Lambdapapillomavirus, Mupapillomavirus, Nupapillomavirus, Omegapapillomavirus, Omikronpapillomavirus, Phipapillomavirus, Pipapillomavirus, Psipapillomavirus, Rhopapillomavirus, Sigmapapillomavirus, Taupapillomavirus, Thetapapillomavirus, Upsilonpapillomavirus, Xipapillomavirus and Zetapapillomavirus.

Papillomaviridae viruses can in particular be the Human papillomavirus (HPV), more particularly HPV16, HPV39, HPV67, HPV68a or HPV53.

Polyomaviridae viruses can in particular be selected from the group consisting of the JC virus (JCV) found in a patient (initials JC), with Hodgkin's lymphoma who suffered from progressive multifocal leukoencephalopathy (PML); the BK virus (BKV) isolated from urine of a kidney transplant patient (initials BK); Merkel cell polyomavirus (MCPyV) associated with Merkel cell carcinoma; and the Trichodysplasia spinulosa-associated polyomavirus (TSPyV).

Polyomaviridae viruses can in particular be selected from the group consisting of the BK virus (BKV) and the Merkel cell polyomavirus (MCPyV).

Members of the Adenoviridae family include members of the genus Mastadenovirus, as for example Mastadenovirus A, Mastadenovirus B, Mastadenovirus C, Mastadenovirus D, Mastadenovirus E, Mastadenovirus F and Mastadenovirus G, which includes human adenoviruses.

They in particular include human adenovirus (HAdV)-A, HAdV-B, HAdV-C, HAdV-D, HAdV-E, and HAdV-F.

Parvoviridae viruses can be selected from the group consisting of Parvovirus, Erythrovirus, Dependovirus, Amdovirus, Bocavirus, Densovirus, Iteravirus, Brevidensovirus and Pefudensovirus.

Parvoviridae viruses are preferably an Erythovirus, and can more particularly be the Human parvovirus B19.

Reoviridae viruses can be selected from the group consisting of Orthoreovirus, Orbivirus, Rotavirus, Coltivirus, Aquareovirus, Cypovirus, Fijivirus, Phytoreovirus, Oryzavirus, Idnoreovirus and Mycoreovirus. Preferably, Reoviridae viruses are selected from Orbivirus, more preferably Kemerovo virus and Orungo virus; and Rotavirus, more preferably from Human rotavirus A.

An Hepevirus of the invention is in particular the Hepatitis E virus.

A Norovirus of the invention can in particular be selected from the group consisting of Human Norovirus saitama and human Norovirus.

An Enterovirus of the invention can in particular be selected from the group consisting of Coxsackievirus, in particular Coxsackievirus A20 and A16; Echovirus ; Enterovirus A71 ; Human rhinovirus; Rhinovirus C and Poliovirus, in particular human poliovirus 2.

A non-enveloped DNA virus of the invention can more preferably be selected from the group consisting of Mastadenovirus A; Mastadenovirus B; Mastadenovirus C; Mastadenovirus D; Mastadenovirus E; Mastadenovirus F; Mastadenovirus G; adenovirus; Human papillomavirus (HPV), in particular HPV16, HPV39, HPV67, HPV68a and HPV53; the BK virus (BKV); the Merkel cell polyomavirus (MCPyV); the Human parvovirus B19; Human rotavirus A; Kemerovo virus; Orungo virus; Hepatitis E; Human Norovirus saitama; human Norovirus; human poliovirus 2; Coxsackievirus A20; Coxsackievirus A16; Rhinovirus C; Human rhinovirus and Enterovirus A71.

A non-enveloped DNA virus of the invention is more preferably an adenovirus, in particular a human adenovirus.

Enveloped RNA viruses can in particular be selected from the group consisting of Filoviridae viruses, Bunyaviridae viruses, Flaviviridae viruses, Paramyxoviridae viruses, Poxviridae viruses, Alphatorquevirus viruses, Anneloviridae viruses, Coronaviridae viruses, Rubella virus, Alphavirus, Paramyxoviridae viruses, Rhabdoviridae viruses, Influenza A virus, Arenavirus, Bunyaviridae viruses, Retroviridae viruses and Hepadnaviridae viruses.

Filoviridae viruses, also called filoviruses, of the invention are in particular selected from the group consisting of Marburg virus, Ebola virus and Ravn virus.

Flaviviridae viruses of the invention are preferably selected from the group consisting of Hepacivirus, Flavivirus, Pegivirus and Pestivirus. They are more preferably selected from Hepacivirus, in particular Hepatitis C virus; and Flavivirus, in particular Dengue virus 4 and Dengue virus 5.

Poxviridae viruses of the invention can in particular be selected from the group consisting of Parapoxvirus, Betaentomopoxvirus, Yatapoxvirus, Cervidpoxvirus, Gammaentomopoxvirus, Leporipoxvirus, Suipoxvirus, Molluscipoxvirus, Crocodylidpoxvirus, Alphaentomopoxvirus, Capripoxvirus, Orthopoxvirus and Avipoxvirus.

Poxviridae viruses of the invention can preferably be selected from the group consisting of an Orthopoxvirus, in particular Cowpox virus, Vaccinia virus, Monkeypoxvirus and Variola virus; and a Parapoxvirus, in particular Orf virus; and a Leporipoxvirus, in particular Myxoma virus; and a Capripoxvirus, in particular Lumpy skin disease virus.

Herpesviridae viruses of the invention can in particular be selected from the group consisting of Iltovirus, Proboscivirus, Cytomegalovirus, Mardivirus, Rhadinovirus, Macavirus, Roseolovirus, Simplexvirus, Scutavirus, Varicellovirus, Percavirus, Lymphocryptovirus and Muromegalovirus.

Herpesviridae viruses of the invention can preferably be selected from Varicellovirus, more preferably Human Herpesvirus 3; Simplexvirus, more preferably Human Herpesvirus 1; Cytomegalovirus, more preferably Human Herpesvirus 5; Roseolovirus, more preferably Human Herpesvirus 6A and Human Herpesvirus 7; and Lymphocryptovirus, more preferably Human Herpesvirus 4.

Annelloviridae viruses of the invention are in particular Alphatorquevirus, preferably Torque teno virus.

Coronaviridae viruses of the invention are in particular Coronavirinae viruses or Torovirinae viruses, preferably Coronavirus, in particular selected from the group consisting of SARS coronavirus sf098, human coronavirus OC43 and transmissible gastroenteritis virus.

Alphavirus of the invention can in particular be selected from the group consisting of Barmah Forest virus, Chikungunya virus, Mayaro virus, O'nyong'nyong virus, Ross River virus, Semliki Forest virus, Sindbis virus, Una virus, Eastern equine encephalitis virus, Tonate virus, Venezuelan equine encephalitis virus and Western equine encephalitis virus.

Alphavirus of the invention is preferably selected from Chikungunya virus and Ross River virus.

Paramyxoviridae viruses of the invention are preferably selected from the group consisting of Avulavirus, Morbillivirus, Aquaparamyxovirus, Henipavirus, Respirovirus, Rubulavirus, Ferlavirus, Pneumovirus and Metapneumovirus.

Paramyxoviridae viruses are preferably selected from the group consisting of Metapneumovirus, in particular human metapneumovirus; Pneumovirus, in particular Human respiratory syncytial virus; Rubulavirus, in particular Mumps virus; and Morbillivirus, in particular Measles virus.

Rhabdoviridae viruses are preferably selected from the group consisting of Lyssavirus, Novirhabdovirus, Ephemerovirus, Perhabdovirus, Tibrovirus, Nucleorhabdovirus, Tupavirus, Vesiculovirus, Sprivivirus, Cytorhabdovirus and Sigmavirus.

Rhabdoviridae viruses are more particularly selected from the group consisting of Lyssavirus, in particular Vesicular stomatitis virus, Piry virus, Isfahan virus and Chandipura virus; and Vesiculovirus, in particular Mokola virus, Rabies virus, Aravan virus and Irkut virus.

Arenavirus of the invention can in particular be selected from the group consisting of Junin virus, Lymphocytic choriomeningitis virus, Lassa virus, Luna virus, Lunk virus, Dandenong virus, Mobala virus and Ippy virus.

Bunyaviridae viruses of the invention can in particular be selected from the group consisting of Batai virus; Hantavirus, in particular Dobrava-Belgrade virus; Nairovirus; Orthobunyavirus; Phlebovirus; and Tospovirus.

Retroviridae viruses of the invention can in particular be selected from the group consisiting of Alpharetrovirus, Betaretrovirus, Deltaretrovirus, Epsilonretrovirus, Gammaretrovirus, Lentivirus and Spumavirus.

Retroviridae viruses of the invention are more preferably selected from the group consisting of Lentivirus, in particular human immunodeficiency virus 1 and human immunodeficiency virus 2; Deltaretrovirus, in particular Human T-lymphotropic virus 1 (HTLV-1) and Human T-lymphotropic virus 2 (HTLV-2); and Alpharetrovirus, in particular Rous sarcoma virus and Rous associated virus.

Hepadnaviridae viruses of the invention are in particular selected from the group consisting of Avihepadnavirus and Orthohepadnavirus, and is more preferably Hepatitis B virus.

An enveloped RNA virus considered in the present invention is preferably selected from the group consisting of Orf virus, Cowpox virus, Vaccinia virus, Monkeypoxvirus, myxoma virus, Lumpy skin disease virus, Variola virus, human herpesvirus 3, human herpesvirus 1, human herpesvirus 5, human herpesvirus 6A, human herpesvirus 7, human herpesvirus 4, Torque teno virus, Dengue virus 4, Dengue virus 5, Hepatitis C virus, SARS coronavirus sf098, Human coronavirus OC43, Transmissible gastroenteritis virus, Ross river virus, Chikungunya virus, Rubella virus, Ebola virus, Marburg virus, Ravn virus, Human metapneumovirus, human respiratory syncytial virus, Mumps virus, Measles virus, Vesicular stomatitis virus, Piry virus, Isfahan virus, Chandipura virus, Mokola virus, Rabies virus, Aravan virus, Irkut virus, Influenza A virus, Junin virus, Lymphocytic choriomeningitis virus, Lassa virus, Luna virus, Lunk virus, Dandenong virus, Mobala virus, Ippy virus, Batai virus, Dobrava-belgrade virus, HIV-1, HIV-2, HTLV-1, HTLV-2, Rous sarcoma virus, Rous associated virus and Hepatitis B virus.

An enveloped RNA virus considered in the present invention is more preferably a filovirus.

More particularly, a virus considered in the present invention can be selected in the group consisting of:
- a non-enveloped DNA virus of the invention can more preferably be selected from the group consisting of Mastadenovirus A; Mastadenovirus B; Mastadenovirus C; Mastadenovirus D; Mastadenovirus E; Mastadenovirus F; Mastadenovirus G; adenovirus; Human papillomavirus (HPV), in particular HPV16, HPV39, HPV67, HPV68a and HPV53; the BK virus (BKV); the Merkel cell polyomavirus (MCPyV); the Human parvovirus B19; Human rotavirus A; Kemerovo virus; Orungo virus; Hepatitis E; Human Norovirus saitama; human Norovirus; human poliovirus 2; Coxsackievirus A20; Coxsackievirus A16; Rhinovirus C; Human rhinovirus and Enterovirus A71; and
- an enveloped RNA virus considered in the present invention is preferably selected from the group consisting of Orf virus, Cowpox virus, Vaccinia virus, Monkeypoxvirus, Variola virus, human herpesvirus 3, human herpesvirus 1, human herpesvirus 5, human herpesvirus 6A, human herpesvirus 7, human herpesvirus 4, Torque teno virus, Dengue virus 4, Dengue virus 5, Hepatitis C virus, SARS coronavirus sf098, Human coronavirus OC43, Transmissible gastroenteritis virus, Ross river virus, Chikungunya virus, Rubella virus, Ebola virus, Marburg virus, Ravn virus, Human metapneumovirus, human respiratory syncytial virus, Mumps virus, Measles virus, Vesicular stomatitis virus, Piry virus, Isfahan virus, Chandipura virus, Mokola virus, Rabies virus, Aravan virus, Irkut virus, Influenza A virus, Junin virus, Lymphocytic choriomeningitis virus, Lassa virus, Luna virus, Lunk virus, Dandenong virus, Mobala virus, Ippy virus, Batai virus, Dobrava-belgrade virus, HIV-1, HIV-2, HTLV-1, HTLV-2, Rous sarcoma virus, Rous associated virus and Hepatitis B virus.

A virus considered in the present invention is more preferably an adenovirus and/or a filovirus.

In another embodiment, a virus considered in the present invention is more preferably a Poxvirus and/or Flavivirus and/or Paramyxovirus.

In a preferred embodiment, the compounds of the present invention can be used in a pharmaceutically acceptable carrier of a composition.

An object of the present invention is thus a composition comprising, in a pharmaceutically acceptable carrier, at least one compound of formula (I), (II) or (III) according to the invention.

In a preferred embodiment, the present invention relates to a composition comprising, in a pharmaceutically acceptable carrier, at least one compound of formula (IIIa).

In a particular embodiment, a composition according to the invention comprises, in addition to the at least one compound of formula (I), (II) or (III) of the invention, at least one additional antiviral compound.

Said at least one additional antiviral compound, different from a compound according to the invention, can in particular be selected from the group consisting of abacavir, acyclovir, adefovir, amantadine, amprenavir, ampligen, arbidol, atazanavir, atripla, balavir, bictegravir, brivudine, cidofovir, cobicistat, combivir, dolutegravir, darunavir, delavirdine, didanosine, docosanol, dolutegravir, edoxudine, efavirenz, elvitegravir, emtricitabine, enfuvirtide, entecavir, ecoliever, famciclovir, fomivirsen, fosamprenavir, foscarnet, fosfonet, ganciclovir, ibacitabine, imunovir, idoxuridine, imiquimod, inidinavir, inosine, lamivudine, letermovir, lopinavir, loviride, maraviroc, moroxydine, methisazone, nelfinavir, nevirapine, nexavir, nitazoxanide, norvir, oseltamivir, penciclovir, peramivir, pleconaril, pritelivir, raltegravir, ribavirin, rilpivirine, rimantadine, ritonavir, saquinavir, sofosbuvir, stavudine, telaprevir, tenofovir, tipranavir, trifluridine, trizivir, tromantadine, truvada, valaciclovir, valganciclovir, vicriviroc, vidarabine, viramidine, zalcitabine, zanamivir, zidovudine, and their combinations thereof.

For example, said at least one additional antiviral compound can be selected among anti-herpetic compounds, such as acyclovir, brivudine, docosanol, famciclovir, foscarnet, idoxuridine, penciclovir, trifluridine, valacyclovir and pritelivir.

For example, said at least one additional antiviral compound can be selected among anti-influenza compounds, such as amantadine, rimantadine, oseltamivir, peramivir and zanamivir.

For example, said at least one additional antiviral compound can be selected among anti-HIV compounds, such as abacavir, amprenavir, doluprenavir, lamivudine, elvitegravir, cobicistat, emtricitabine, tenofovir, efavirenz, rilpivirine bictegravir, dolutegravir, raltegravir, abacavir and zidovudine.

For example, said at least one additional antiviral compound can be selected among anti-cytomegalovirus compounds, such as ganciclovir, letermovir, foscarnet, cidofovir, valaciclovir and valganciclovir.

For example, said at least one additional antiviral compound can be selected among anti-adenovirus compounds, such as cidofovir and ribavirin.

The routes of administration and dosage vary depending on a variety of parameters, for example depending on the individual's condition, the type of infection and the severity of infection to be treated or of compound(s) of the invention and of the other antiviral agents used. The compound according to the invention and the composition according to the invention are especially capable of being administered to an individual in dry, solid (in particular cachet, powder, capsule, pill, granule, suppository, or tablet polymer capsule, specifically accelerated release tablet, enteric tablet or prolonged-release tablets), under gelatinous form, or as a solution, or a liquid suspension (in particular syrup, injectable solution, or oral infusible, microvesicles, liposomes).

These compounds can also be in the form of doses in dry form (powder, lyophilizate, etc.) for reconstitution at the time of use using appropriate diluents known to the man skilled in the art.

Depending on their dosage form, a composition of the invention may be administered enterally, parenterally (intravenously, intramuscularly or subcutaneous), transdermally (or percutaneous or transdermal), cutaneously, orally, mucosally, in particular transmucosally, buccally, nasally, ophthalmically, otologically (in the ear), esophageally, vaginally, rectally, or intragastrically, intracardiacally, intraperitoneally, intrapulmonaryly or intratracheally.

Furthermore, the compound of the invention or the composition of the invention may be packaged for administration as a single dose (single dose) or multiple (multidose).

To enhance the effects of treatment, it is possible to proceed with an administration in the form of several successive administrations, repeated at one or more occasions after a particular time interval. One can, for example, carry out several administrations per day or per week.

The amount of active ingredient administered to an individual is in a therapeutically effective amount.

A "therapeutically effective amount" is an amount sufficient to produce a significant effect, especially bring a significant benefit to said individual as part of an administration for the prophylaxis or treatment as defined previously.

A therapeutically effective amount is an amount for which the beneficial effects outweigh any toxic or detrimental effect of or ingredient(s) active(s). The therapeutically effective amount will vary depending on factors such as the state of infection, age, sex or weight of the individual.

Dosage regimens may be adjusted to obtain an optimum therapeutic effect.

More specifically, a therapeutically effective amount of compound of structure I of the invention may be between 40 mg/day and 1600 mg/day, in particular between 80 mg/day and 1200 mg/day, more particularly between 100 mg/day and 800 mg/day, preferably between 200 mg/day and 500 mg/day, administered for example in 1 to 3 doses.

The present invention is illustrated by the following examples, given purely for illustrative purposes, with reference to the following figures.

### Example 1: Preparation of the compound of formula (IIIa)

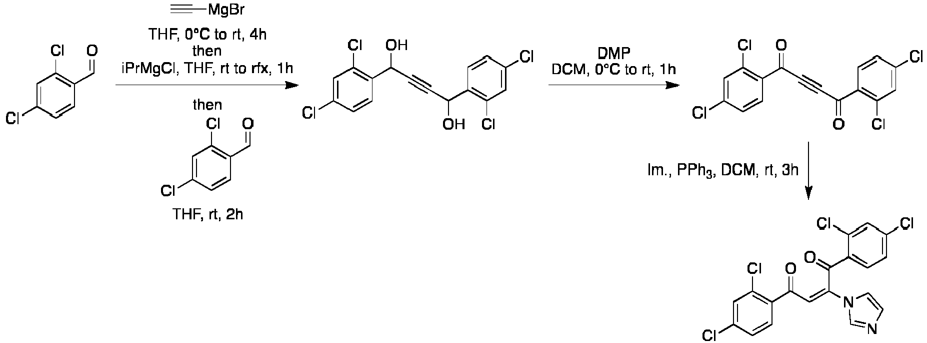

### 1,4-Bis(2,4-dichlorophenyl)but-2-yne-1,4-diol

To a stirred solution of 2,4-dichlorobenzaldehyde (7.3 g, 41.7 mmol) in dry THF (85 mL) under inert atmosphere was added a 0.5 M solution of ethynylmagnesium bromide in THF (100 mL, 50 mmol, 1.2 eq.) at 0 °C. The resulting mixture was stirred at this temperature for 15 minutes then at room temperature for 4 h before addition of an aqueous NH₄Clsat. solution. The reaction mixture was extracted with EtOAc (three times) and the combined organic extracts were washed with brine, dried over MgSO₄, filtered and the filtrate concentrated under reduced pressure. The crude 1-(2,4-dichlorophenyl)prop-2-yn-1-ol was dissolved in dry THF (100 mL) and a 2 M solution of isopropylmagnesium chloride in THF (42 mL, 84 mmol, 2 eq.) was slowly added under inert atmosphere. The reaction mixture was then heated at reflux for 1 h then cooled to room temperature and a solution of 2,4-dichlorobenzaldehyde (7.3 g, 41.7 mmol, 1 eq.) in dry THF (50 mL) was added. After 2 h at room temperature aqueous NH₄Clsat. solution was added and the reaction mixture extracted with Et₂O (3 times). The combined organic extracts were washed with brine, dried over MgSO₄, filtered and the filtrate concentrated under reduced pressure. The expected product was precipitated by addition of cold DCM and isolated by filtration affording of the expected compound as a white powder (11.4 g, 73% yield).
¹H NMR (DMSO-*d₆*, 300 MHz): δ 7.68 (dd, *J* = 8.4, 2.0 Hz, 2H), 7.60 (m, 2H), 7.48 (dd, *J* = 8.4, 2.0 Hz, 2H), 6.34 (m, 2H), 5.58 (m, 2H).
¹³C NMR (DMSO-d₆, 75 MHz): δ 138.2, 133.1, 132.2, 129.4, 128.7, 127.5, 84.4, 59.4.

### 1,4-Bis(2,4-dichlorophenyl)but-2-yne-1,4-dione

To a stirred solution of 1,4-bis(2,4-dichlorophenyl)but-2-yne-1,4-diol (5 g, 13.3 mmol) in DCM (50 mL) was added Dess-Martin periodinane (12.4 g, 29.2 mmol, 2.2 eq.) at 0 °C. The reaction mixture was stirred at this temperature for 15 minutes then at room temperature for 1 h. The reaction mixture was washed with a 1:1 mixture of aqueous NaHCO₃sat./Na₂S₂O₃sat. solution. The aqueous phase was then extracted with DCM (3 times) and the combined organic extracts washed with brine, dried over MgSO₄, filtered and the filtrate concentrated under reduced pressure. Purification by recristallisation in EtOAc afforded of the expected compound as a white solid (3.25 g, 66% yield).
¹H NMR (CDCl₃, 300 MHz): δ 8.04 (d, *J* = 8.5 Hz, 2H), 7.55 (d, *J* = 2.0 Hz, 2H), 7.43 (dd, *J* = 8.5, 2.0 Hz, 2H).
¹³C NMR (CDCl₃, 75 MHz): δ 173.6, 140.8, 135.3, 134.1, 132.2, 131.8, 127.6, 87.0.

### (E)-1,4-Bis(2,4-dichlorophenyl)-2-(1H-imidazol-1-yl)but-2-ene-1,4-dione

To a stirred solution of PPh₃ (705 mg, 2.7 mmol, 1 eq.) and imidazole (183 mg, 2.7 mmol, 1 eq.) in DCM (5.5 mL), under inert atmosphere, was slowly added a solution of 1,4-bis(2,4-dichlorophenyl)but-2-yne-1,4-dione (1 g, 2.7 mmol) in DCM (2.7 mL). The reaction mixture was stirred at room temperature for 3 h then concentrated under reduced pressure. The expected product was isolated after several flash column chromatography on silica gel (acetone/PE (20% → 80%); Et₂O/DCM (5 → 20%); EtOAc/DCM (5% → 25%) as a clear foam (113 mg, 10% yield).
¹H NMR (CDCl₃, 300 MHz): δ 7.94 (d, *J* = 8.5 Hz, 1H), 7.79 (s, 1H), 7.49 - 7.43 (m, 3H), 7.38 (dd, *J* = 8.5, 2.0 Hz, 1H), 7.32 (dd, *J* = 8.3, 2.0 Hz, 1H), 7.23 (t, *J* = 1.4 Hz, 1H), 7.21 (s, 1H), 7.06 (s, 1H).
¹³C NMR (CDCl₃, 300 MHz): δ 188.8, 188.0, 145.9, 140.5, 139.1, 136.1, 134.8, 133.4, 132.9, 132.1, 131.7, 131.5, 130.5, 128.0, 127.9, 117.1, 114.4.
HRMS ESI [M+H]⁺ Calc. for C₁₉H₁₁N₂O₂Cl₄ 438.9575; Found 438.9570.

### Example 2: Inhibition of adenoviral propagation by the compound of formula (IIIa) and comparison of its activity compared to a lead compound: Cidofovir™

The compound of formula (IIIa) according to the invention has been tested for its anti-adenoviral activity.

Adenoviral propagation was quantified by fluorescent plaque forming assay with GFP expressing HAd-C5 vector in the presence of the compound as a measure of virus spread through multiple rounds of infection.

### a) Method

HEK293 cells were infected with 0.1 physical particles per cell (pp/cell) of E1-deficient GFP expressing HAd-C5-GFP vector for 24 hours followed by removal of the inoculums and overlay with 1% agarose supplemented with vehicle only (DMSO) or 5 µM of the compound of formula (IIIa).

Six days after infection, plaque sizes were measured by epifluorescence microscopy using Image J.

Cidofovir™, the standard clinical drug used against adenovirus infections in the clinic, is also included in this test.

The compounds were used at a concentration of 5 µM. Cidofovir was in particular used at even higher concentration, i.e. 10 and 20 µM.

DMSO was used as negative control.

### b) Statistics

Statistical analysis was carried out using unpaired Student t-test.

### c) Results

### The results obtained are illustrated in Figure 1.

Compared to the DMSO negative control, the compound of formula (IIIa) demonstrates an effective anti-adenoviral activity.

Moreover, this compound demonstrates an increased activity compared to the lead compound Cidofovir.

In contrast, Cidofovir did not show significant inhibition even at highest non-cytotoxic concentrations (10 µM and 20 µM).

Accordingly, the inventors identified a novel class of compounds having a strong anti-viral activity.

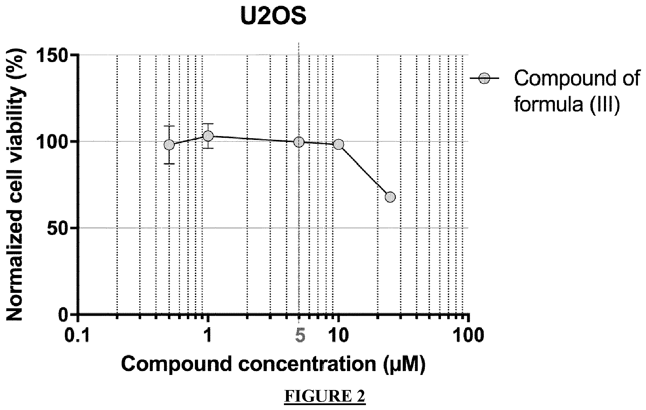

Moreover, the compound of formula (IIIa) showed an absence of cytotoxicity at a concentration of 5 µM on U2OS cells (see Figure 2).

To determine said cytotoxicity, U2OS cells were treated for 36 h with 250 nM to 50 µM of the compound of formula (IIIa). Cell viability in the presence of the candidate drug was monitored using the CellTiter-Glo® Luminescent Cell Viability Assay (Promega) according to the manufacturer's instructions. Cell viability was normalized to the mean of the signal obtained in presence of DMSO alone. The mean values were determined from a triplicate experiment and error bars are SD.

### Example 3: The compound of formula (IIIa) of the invention interacts with the NEDD4 peptide

The compound of formula (IIIa) of the invention is now shown herein to be active against adenovirus and filovirus.

It was previously reported that the NEDD4/[LP]PxY interaction contributes to efficient entry of non-enveloped adenoviruses (Wodrich et al. ; 2010; A Capsid-Encoded PPxY-Motif Facilitates Adenovirus Entry. PLoS Pathog. 6, e1000808). Also, NEDD4/[LP]PxY interaction has been exploited as drug targets for enveloped RNA viruses (Han et al.; Small-Molecule Probes Targeting the Viral PPxY-Host Nedd4 Interface Block Egress of a Broad Range of RNA Viruses. J. Virol. 88, 7294-7306 ; 2014).

Accordingly, the inventors sought for the mechanism of action responsible for the antiviral activity of the compounds of the invention by investigating their ability to modulate the interaction between NEDD4 and peptides bearing a [LP]PxY motif.

To this end they have used solution nuclear magnetic resonance spectroscopy (NMR) to obtain structural information. They have synthesized a peptide corresponding to the 45 amino acids of WW3-domain of NEDD4 and for which an NMR solution structure bound to a PPxY containing peptide from EBOV was previously obtained (PDB ID: 2KQ0). Subsequently NMR analysis was performed in absence and presence of the compound of formula (IIIa).

### a) Methods

**NMR.** The 1D and 2D 1H NMR experiments (Total Correlation Spectroscopy (TOCSY), Nuclear Overhauser Effect Spectroscopy (NOESY) Correlation Spectroscopy (COSY) and the and 2D 1H-13C heteronuclear NMR experiments Heteronuclear Single Quantum Coherence (HSQC) and Heteronuclear Single Quantum Coherence-Nuclear Overhauser Effect Spectroscopy (HSQC-NOESY) were performed at 850.13 MHz on a Bruker 850 MHz instrument equipped with an UltraShield Plus magnet and a triple resonance cryoprobe with gradient unit. The 2D NMR experiments were performed at 300 K without spinning with mixing times of 110 ms for the TOCSY experiments and 250 ms for the NOESY and HSQC-NOESY experiments, respectively.

**Docking studies.** The crystallographic coordinates of human NEDD4 third WW domain complexed with Ebola Zaire Virus Matrix protein VP40 derived peptide (PDB id 2KQ0). The peptide ligand was removed and the protein was prepared using MOE 2015.10 [Molecular Operating Environment (MOE). 2015, Chemical Computing Group: Montreal, Canada.]. To this end, hydrogen atoms were added using MOE's protonate 3D facility with the dielectric constants set to 3 inside the protein and 80 for the solvent (water). The temperature was set to 310 K, the salt concentration to 0.2 M and the pH to 7.0. The Lennard-Jones 12-6 potential was used for simulating van der Waals interactions, whereas electrostatic interactions were simulated according to the GB/VI model (Gilson, M.K. (1993). Multiple-site titration and molecular modeling: two rapid methods for computing energies and forces for ionizable groups in proteins. Proteins 15, 266-282.; Labute, P. (2008). The generalized Born/volume integral implicit solvent model: estimation of the free energy of hydration using London dispersion instead of atomic surface area. J. Comput. Chem. 29, 1693-1698).

Cutoff distances were set to 15 Å and 10 Å for the electrostatic and van der Waals interactions, respectively. The structure was energy minimized using the AMBER12 force field (Case et al. 2012) to within an rms gradient of 0.01 kcal.mol⁻¹.Å⁻¹. Throughout, all systems were surrounded by a distance dependent dielectric model of bulk water. The resulting protein model was used in the docking studies.

Three peptide ligands were obtained from the corresponding crystal structures, 2KQ0, 2LAJ, and 2MPT, whereas the two other peptides (DEPPSYEE and DEPGAAEE) were built in MOE and prepared following the described methodology. The compound of formula (IIIa) was built in MOE and energy minimized using the AMBER12 forcefield to within an rms gradient of 0.001 kcal.mol⁻¹.Å⁻¹. The peptide ligand was docked into the human NEDD4 protein, employing the protein-protein docking module in MOE, selecting a pocket in NEDD4 as receptor (the cavity formed by two antiparallel β-sheets) and the compound of formula (IIIa) as ligand. The protein-protein docking facility generates different poses using FFTs (Fast Fourier Transforms) followed by all atom minimization. Finally, the small ligand was computationally docked in the same pocket of NEDD4 using the alpha triangle placement methodology with affinity ΔG as scoring function as implemented in MOE. In the docking studies, flexible ligand structures were generated using a Monte Carlo algorithm, whereas the receptor was held fixed according to the minimized geometry.

**Molecular Dynamics simulations.** All molecular dynamics simulations of the peptide - NEDD4 complexes were performed using the YASARA Structure program (Krieger, 2004-2016, YASARA). The ligand-model complexes as obtained from the docking studies were energy minimized to within an rms gradient of 0.1 kcal mol⁻¹ Å⁻¹ using the Amber ff03 molecular mechanics force field (Duan et al. (2003). A point-charge force field for molecular mechanics simulations of proteins based on condensed-phase quantum mechanical calculations. J. Comput. Chem. 24, 1999-2012).

The models were then solvated and minimized in a box of water extending 10 Å from the nearest atoms of the complex, giving approximately 30 000 atoms in each simulation box. Taking pKa values into account, protonation states were assigned at physiologic pH 7.4 (Krieger et al. (2012). Assignment of protonation states in proteins and ligands: combining pKa prediction with hydrogen bonding network optimization. Methods Mol. Biol. Clifton NJ 819, 405-421).

In order to achieve physiological salt concentration, water molecules were randomly replaced by sodium or chloride ions and the solvent density adjusted to 0.997 g L⁻¹, followed by Molecular Dynamics (MD) simulation and final energy minimization step. The MD simulation was performed for 50 ns using periodic boundary conditions and the NVT canonical ensemble at 298 K. The simulation was carried out using the multiple time steps 2.5 fs for intermolecular forces and 1.25 fs for intramolecular ones. The particle mesh Ewald (PME) method was used for long-range electrostatics with a cutoff of 7.86 Å (Linse and Linse (2014) Tuning the smooth particle mesh Ewald sum: application on ionic solutions and dipolar fluids. J. Chem. Phys. 141, 184114). Trajectory snapshots were sampled every 100 ps during the MD simulation.

### b) Results

The NMR analyses showed that the compound of formula (IIIa) interacts with the NEDD4 peptide. When equimolar amounts of the compound of formula (IIIa) was added to the NEDD4 peptide the chemical shift values of the imidazole ring of the compound of formula (IIIa) changed significantly when compared with that of the pure compound of formula (IIIa) dissolved in the same solvent at the same concentrations, whereas the chemical shift values of the remaining aromatic hydrogens and carbon atoms mainly remained unchanged. The NMR data suggested binding between the compound of formula (IIIa) and the NEDD4 peptide involved the imidazole ring and amino acids W39 and F28.

Exploiting this information, a docking study was performed with an active site bias derived from the NMR data on the same structure model (2KQ0) we used for the peptide/protein interaction studies. The compound of formula (IIIa) efficiently docked on the exposed [LP]PxY peptide binding surface of the WW-domain with the imidazole ring inserting into a cavity formed by W39 and F28 thus confirming the NMR data and providing a structural rational for the interaction.

Importantly, when the docking predictions for the viral DEPPSYEE peptide and the compound of formula (IIIa) were superimposed on the NEDD4 structure the imidazole ring occupied the same cavity that was occupied by the essential second Proline of the [LP]PxY motif while one of the compound of formula (IIIa) aromatic rings occupied the same binding interface as the essential Tyrosine of the motif.

In conclusion, NMR spectroscopy analysis using a synthetic peptide encoding the WW3 domain of NEDD4 showed direct interaction between the imidazole ring of the compound of formula (IIIa) and a binding pocket on the exposed substrate binding surface of WW3. Most importantly *in silico* docking analysis suggested that the compounds of formula (I) occupied an essential binding sites for the [LP]PxY motif, thus acting as allosteric competitor for the binding of [LP]PxY motif containing substrates.

The NMR based structure model also provides evidence that imidazole-derivatives with higher affinity and specificity can be generated via targeted rational drug design; which thus validates the broad antiviral effect of the compounds of formula (I).

### Example 4: Determination of the binding constant (K_{D}) for the compound of formula (IIIa) to the WW3 domain of NEDD4 peptide and comparison with imidazole using MicroScale thermophoresis (MST)

For the MicroScale Thermophoresis (MST) experiments 1 mg of each FAM-labeled WW3_Nedd.4.1 (PSEIEQGFLPKGWEVRHAPNGRPFFIDHNTKTTTWEDPRL KIPAH = SEQ ID NO: 1) and WW3_Nedd.4.2 peptide (GAMEQSFLPPGWEMRIAPNG RPFFIDHNTKTTTWEDPRLKFPVH = SEQ ID NO: 2) (FAM = Fluorescein amidite) was used at >80% purity.

MicroScale thermophoresis binding experiments were carried out with final 100 nM FAM-labelled WW3_Nedd.4.1 and WW3_Nedd.4.2 in binding buffer (1x PBS pH 7.4, 5 % DMSO, 0.1 % Pluronic F-127). The concentration range of the compound of formula (IIIa) is indicated here-after:

**Table 1**

| **Ligand** | **Maximal concentration in nM** | **Minimal concentration in nM** |
|---|---|---|
| **Compound of formula (IIIa)** | 15 625 | 0.476 |
| **Imidazole (control)** | 10 000 000 | 305.18 |

The MST analyses were performed at 40 % MST power, 100 % LED power in standard capillaries on a Monolith NT.115 blue/green device at 25°C (NanoTemper Technologies, Munich, Germany) in duplicate technical runs in the same capillary. Thermophoresis + Tjump was analysed. The recorded fluorescence was normalised to fraction bound (0 = unbound, 1 = bound), and processed using the GraphPad Prism® 6 software and fitted using the KD fit formula derived from the law of mass action.

### Results

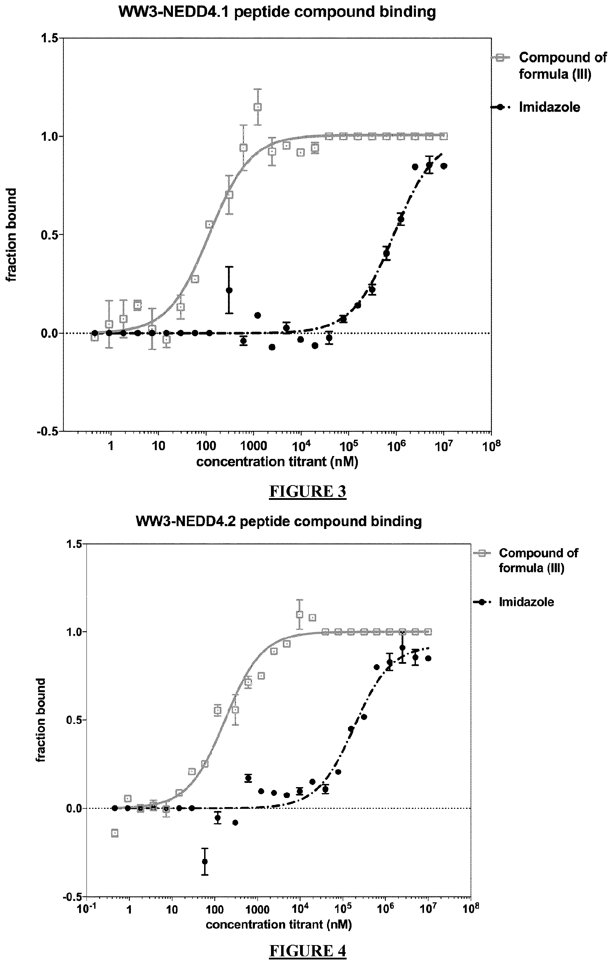

The results obtained are illustrated in Figures 3 and 4.

The affinities measured are represented in the following Table 2:

**Table 2**

| **Ligand** | **WW3-NEDD 4.1 peptide K_{D} (nM)** | **WW3-NEDD 4.2 peptide K_{D} (nM)** |
|---|---|---|
| **Compound of formula (IIIa)** | 58 +/- 22 | 120 +/- 39 |
| **Imidazole (control)** | 88922 +/- 28409 | 90429 +/- 38643 |

The compound of formula (IIIa) has a 100-1000 fold higher affinity to the WW3 peptide domain target compared to the Imidazole control.

The compound of formula (IIIa) identified in this invention binds with low nanomolar range confirming specific peptide binding.

### Example 5: the compound of formula (IIIa) modulates ubiquitylation of PPxY bearing substrates in vitro and in cells

### a) Method

Stably expressing NEDD4.2-GFP cells were transfected with an expression vector encoding RFP-tagged protein VI WT (including the PPxY targeted by NEDD4 ubiquitin ligases through WW-domains, which are the target of the compounds) or protein VI M1 (with the PPxY targeted by NEDD4 ubiquitin ligases through WW-domains mutated to PGAA) and treated with increasing amounts of the compound of formula (IIIa) for 2h. RFP stands for Red Fluorescent Protein.

Protein VI was immunoprecipitated from drug treated cells using RFP-trap and analyzed by western blotting with anti-ubiquitin antibodies.

For *in vitro* ubiquitylation reactions, purified Adenovirus particles were treated with mild heat to release protein VI as previously described in Wodrich et al. (2010 - PLoS Pathog. 6, e1000808), and *in vitro* ubiquitylation reactions were supplemented with recombinant NEDD4.2 and DMSO or the compound of formula (IIIa) and carried out for 1h at 30°C or left on ice.

### b) Results

Samples were analyzed by western blot using anti-protein VI antibodies described in Martinez et al., J Virol. 2015 Feb;89(4):2121-35.

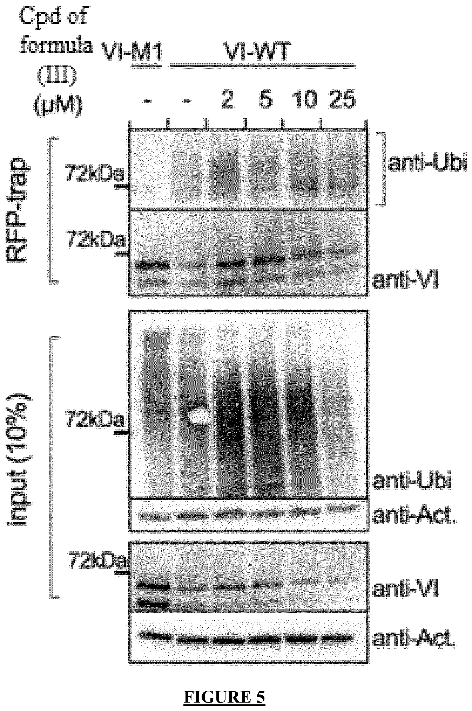

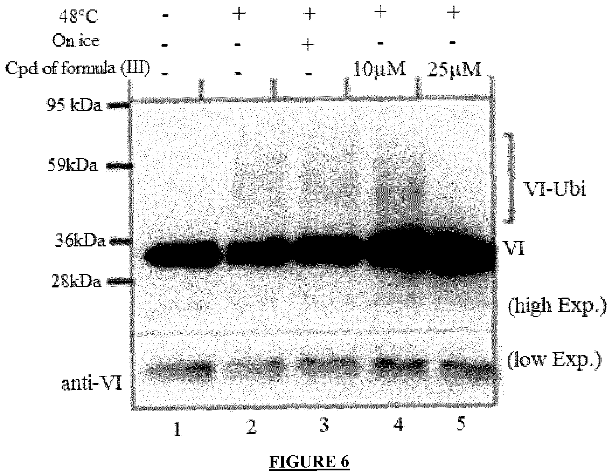

The results are shown in Figures 5 and 6.

They show that ubiquitylation of protein VI was PPxY-dependent and that increasing drug concentrations of the compound of formula (IIIa) led to a shift in the protein VI ubiquitylation patterns, demonstrating that higher concentrations of the compound of formula (IIIa) gradually impaired ubiquitylation of protein VI.

Ubiquitylation of protein VI using recombinant NEDD4.2 revealed that the compound of formula (IIIa) also prevented protein VI ubiquitylation *in vitro* at high concentrations (25 µM). Accordingly, the compound of formula (IIIa) impairs NEDD4.2 mediated ubiquitylation of the viral PPxY bearing substrate in a dose dependent manner.

### Example 6: the compound of formula (IIIa) relocalizes NEDD4 ligases in cells and prevent their association with incoming adenoviruses

### a) Method

U2OS cells (ATCC® HTB-96™) (*Homo sapiens* bone osteosarcoma) stably expressing low levels of GFP-NEDD4.1 or GFP-NEDD4.2 were generated through single copy flp-FRT integration (Bosse et al., Methods Mol Biol. 2013;1064:153-69.) and treated with the compound of formula (IIIa) or the control DMSO.

GFP-NEDD4.2 expressing cells were infected in absence or presence of 10µM of the compound of formula (IIIa) with fluorescently labelled viruses and association between viruses and NEDD4.2-GFP was quantified using fluorescence microscopy.

### b) Results

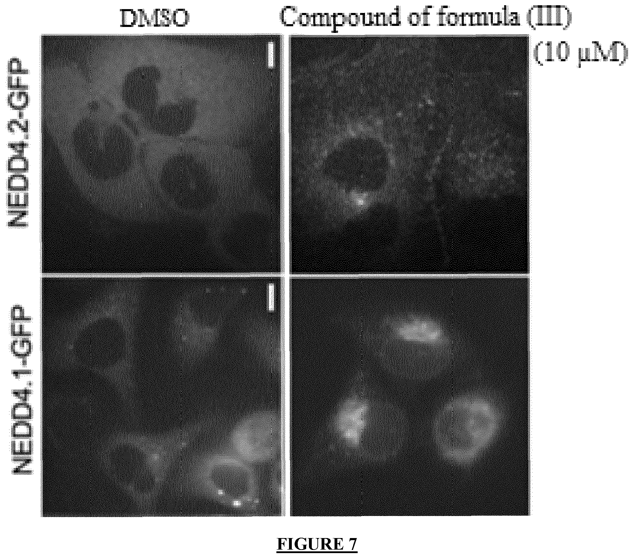

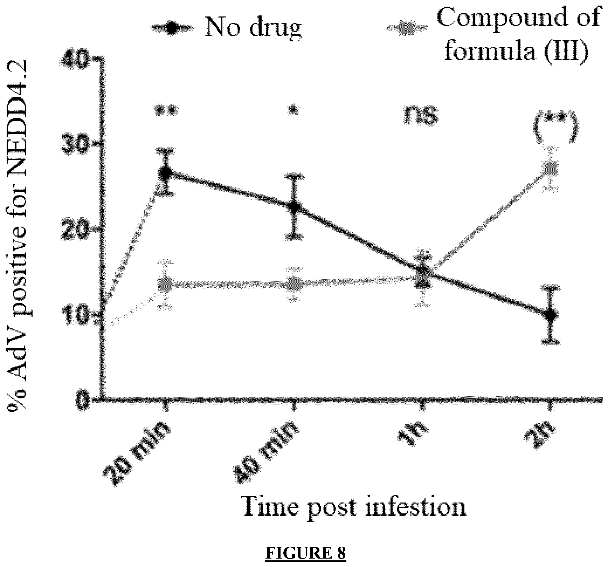

### The results are presented in Figures 7 and 8.

NEDD4.1 became largely immobile and relocated to a perinuclear region upon the compound of formula (IIIa) treatment. NEDD4.2 was mainly cytosolic in untreated cells and accumulated strongly at cell-cell-contact sites as well as in a distinct perinuclear region upon treatment with the compound of formula (IIIa).

Incoming AdV particles associated and co-trafficked with NEDD4.2 within 15-30 min p.i. Kinetics of NEDD4.2 trafficking coincided with protein VI release and membrane penetration (Montespan et al., PLoS Pathog. 2017 Feb 13;13(2):e1006217). In contrast, the compound of formula (IIIa) treatment prevented the recruitment of NEDD4.2 at similar time points.

Accordingly, the compound of formula (IIIa) impairs normal NEDD4.2 localization in uninfected cells and prevents efficient NEDD4.2 association with incoming viruses.

### Example 7: the compound of formula (IIIa) decreases or increases

### ubiquitylation of cellular proteins in cells depending of its concentration

### a) Method

U2OS Cells were treated for 24h with two different concentrations of the compound of formula (IIIa) (2µM and 10µM) and were subjected to Mass Spec. analysis following diGly peptide enrichment to obtain changes in the Ubiquitin proteome.

Briefly, cells were collected following treatment (as above) and protein extracts generated using acetone precipitation. Total proteins were trypsin-digested and the resulting peptides purified through anti di-Gly affinity chromatography prior being sequenced by LC-MS/MS.

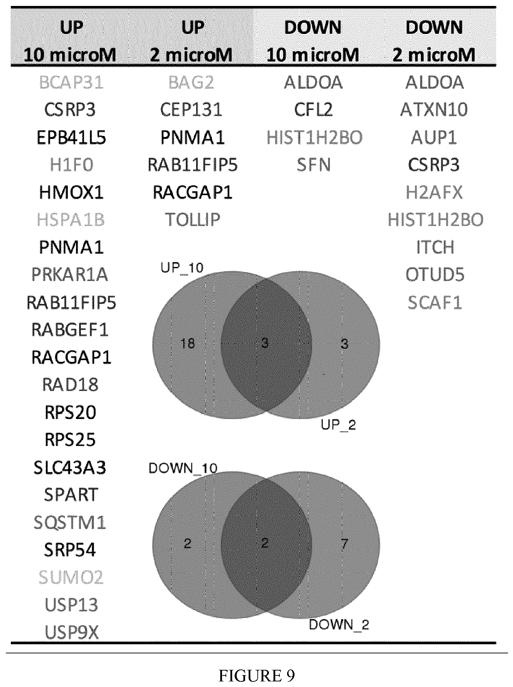

The Figure 9 illustrates the proteins with significant changes in ubiquitylation upon treatment with the compound of formula (IIIa).

### b) Results

### The results obtained are represented in Figure 10.

It is observed that treatment of cells with the compound of formula (IIIa) changes the global cellular protein ubiquitylation patterns.

At low concentration of the compound of formula (IIIa) such as 2µM protein ubiquitylation is reduced or increased for several cellular proteins when compared to DMSO treated control cells, while higher concentrations of this compound, such as 10µM, preferentially increases ubiquitylation of several cellular proteins when compared to DMSO treated control cells.

## Claims

1. A compound of formula (I): 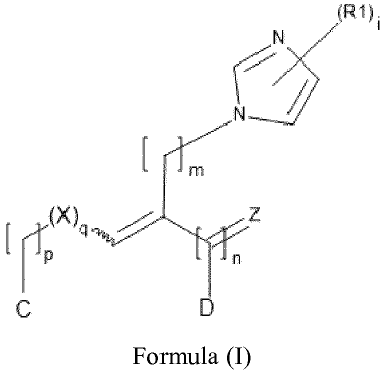 wherein:
q is 0 or 1;
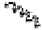 represents a single bond;
X, when q is 1, is selected from the group consisting of -S-, -O-, -C(O)- and -CH₂-;
C and D, independently, represent a non-substituted aromatic ring or an aromatic ring substituted by one or more substituents independently selected from the group consisting of a halogen atom, a C₁-C₄ alkyl group, a C₁-C₄ alkoxy group, a hydroxyl group, a -NO₂ group, a -NR₂R₃ group and a -CN group, with R₂ and R₃ being independently selected from a hydrogen atom and a C₁-C₄ alkyl group;
Z represents O or S;
m and p, independently, are 0, 1 or 2;
n is 0 or 1;
i is 0, 1, 2 or 3; and
R1 independently represents a halogen atom or a C₁-C₄ alkyl group;
or one of its pharmaceutically acceptable salts;
said compound of formula (I) being in all the possible racemic, enantiomeric and diastereoisomeric isomer forms.

2. The compound according to claim 1, wherein q is 1.

3. The compound according to claim 1 or 2, wherein q is 1 and X represents a -C(O)- group.

4. The compound according to anyone of the preceding claims, wherein C and D, independently, represent a phenyl ring, in particular a phenyl ring substituted with one or two halogen atom(s), preferably one or two chlorine atom(s).

5. The compound according to anyone of the preceding claims, wherein m and p are independently 0 or 1.

6. The compound according to anyone of the preceding claims, wherein m and p are both 0.

7. The compound according to anyone of the preceding claims, wherein n is 1 and Z preferably represents O.

8. The compound according to anyone of the preceding claims, wherein i is 0.

9. The compound according to claim 1, wherein the compound is a compound of formula (II): 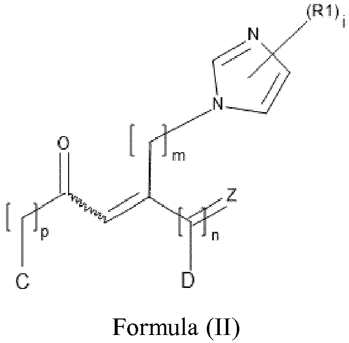
wherein C, D, Z, 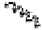 , m, n, p, i and R1 are as defined in the compound of Formula (I) in claim 1;
or one of its pharmaceutically acceptable salts;
said compound of formula (II) being in all the possible racemic, enantiomeric and diastereoisomeric isomer forms.

10. The compound according to claim 9, wherein n is 1 and Z represents O.

11. The compound according to anyone of the preceding claims, wherein the compound is the compound of formula (III): 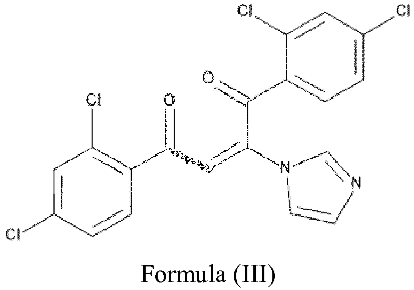
wherein 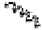 represents a single bond;
or one of its pharmaceutically acceptable salts;
said compound being in all the stereoisomeric forms.

12. The compound according to claim 11, wherein the compound is of formula (IIIa):

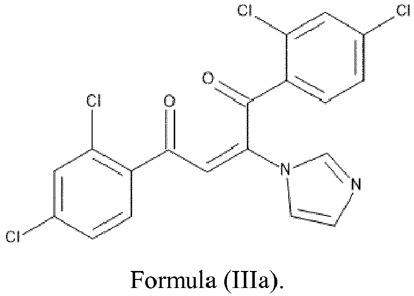

13. A composition comprising, in a pharmaceutically acceptable carrier, at least one compound of formula (I), (II) or (III) as defined in anyone of claims 1 to 12.

14. A compound according to anyone of claims 1 to 12, or a composition according to claim 13, for its use in the prevention and/or treatment of viral infections by a non-enveloped DNA virus and/or an enveloped RNA virus.

15. The compound or composition for use according to claim 14, wherein it is for use in the prevention and/or treatment of viral infections by an adenovirus and/or a filovirus, preferably by an adenovirus.
